# EUROPEAN PATENT SPECIFICATION

(11) **EP 2 884 281 B1**
(45) Date of publication and mention of the grant of the patent: **06.06.2018**
(21) Application number: 14197735.5
(22) Date of filing: 12.12.2014
(51) Int. Cl.: G01N 33/573, G01N 33/84, C12Q 1/00

(54) **Reagent kit, assay method, microfluidic device, and assay apparatus**
Reagenzienkit, Testverfahren, mikrofluidische Vorrichtung und Testvorrichtung
Kit de réactif, procédé d'analyse, dispositif microfluidique et appareil d'analyse

(30) Priority: 13.12.2013 KR 20130155890; 14.04.2014 US 201461979222 P
(43) Date of publication of application: 17.06.2015
(73) Proprietor: Samsung Electronics Co., Ltd., Suwon-si, Gyeonggi-do 443-742 (KR)
(72) Inventor: Shimayama, Takashi, Seoul (KR); Kim, Kui Hyun, Gyeonggi-do (KR); Kang, Ji Yun, Gyeonggi-do (KR); Kim, Soo Hong, Gyeonggi-do (KR); Park, Sung Joon, Gyeonggi-do (KR)
(74) Representative: Grünecker Patent- und Rechtsanwälte PartG mbB

(56) References cited:
- EP-A2- 0 286 039
- US-A- 4 012 285

## Description

### BACKGROUND

### 1. Field

Compositions, methods, and apparatus consistent with exemplary embodiments relate to a reagent kit used to determine the concentration of a target material included in a sample and including a reagent composition, a method of testing a sample using the same, a microfluidic device in which reaction between a sample and a reagent occurs, and a test apparatus for testing a sample based on the reaction occurring in the microfluidic device.

### 2. Description of the Related Art

Recently, small-scale automated equipment capable of rapidly analyzing samples has been developed in a variety of fields, such as environmental monitoring, food inspection, medical diagnosis, and the like.

In particular, in medical diagnosis, a concentration of a target material contained in a sample may be determined by measuring, the amount of an activated enzyme through an enzyme reaction where an enzyme that is activated by a target material acts as a catalyst. Other materials involved in enzyme activation in addition to a target material may be present in a sample and thus, to enhance the reliability and accuracy of the determination, a method which allows minimizing or removing the impacts of other materials than a target material on the enzyme reaction or the measurement of the enzyme amount, is needed.

EP 0 286 039 A2 concerns a process and a reagent for the determination of ions in fluids, wherein the influence of these ions on the activity of an enzyme is measured. The ions for example are sodium, potassium, calcium, magnesium, manganese, lithium, lead, zinc, copper, iron or other heavy metals or non-metallic ions comprising chloride, bicarbonate, protons, ammonium substances that give rise to ammonium. The enzymes which are used may be for example a transferase, a hydrolase, an oxidoreductase or a lyase. An essential part of the invention is a method to exclude interferences by ions by masking the interfering ions with a bindig agent.

According to US 4,012,285 A, isoenzyme patterns of an enzyme in biological fluids, extracts, and/or excretions are quantitatively determined by precipitating at least 90% of an isoenzyme antigen with a corresponding heterologous antiserum and comparing the remaining enzyme activity with the total activity with the use of a conventional agent for enzyme activity determination

### SUMMARY

Therefore, according to an embodiment, a reagent composition that may enhance reliability of measurement results by excluding effects of other materials than a target material on the measurement results by controlling the concentration of the target material present in a sample, a method of testing a sample, a microfluidic device, and a test apparatus are described.

Additional embodiments will be set forth in part in the description which follows and, in part, will be obvious from the description, or may be learned by practice of various embodiments.

In accordance with one aspect, a method of testing a sample to determine a concentration of a target material included in the sample includes mixing the sample with reagent 1 including an enzyme activated by the target material and at least one substrate, reaction of which is catalyzed by the activated enzyme, to induce reaction between the sample and the reagent 1, mixing the sample with reagent 2 including an enzyme activated by the target material, at least one substrate, reaction of which is catalyzed by the activated enzyme, and a capturing material specifically capturing the target material, to induce reaction between the sample and the reagent 2, measuring characteristics obtained by the reaction between the sample and the reagent 1 and characteristics obtained by the reaction between the sample and the reagent 2, calculating a difference value between the measured characteristics, and determining the concentration of the target material included in the sample using the calculated difference value.

The method may further include pre-storing a relationship between the difference value and the concentration of the target material.

The determining may include determining the concentration of the target material included in the sample by substituting the calculated difference value into the pre-stored relationship between the difference value and the concentration of the target material.

The sample may be a biological sample, and the target material may be at least one of electrolyte ions present in the biological sample.

The target material may be at least one of magnesium ions (Mg²⁺), calcium ions (Ca²⁺), potassium ions (K⁺), sodium ions (Na⁺), and chlorine ions (Cl⁻).

The capturing material may be at least one selected from the group consisting of an amino carboxylic acid, a phosphonic acid, a quinoline, a crown ether, a calix-arene, a kryptofix, ehylenediaminetetraacetic acid (EDTA), thio-urea, and porphyrin.

In accordance with another aspect, a reagent composition includes an enzyme activated by a target material present in a sample, at least one substrate, reaction of which is catalyzed by the activated enzyme, and a capturing material specifically capturing the target material.

The capturing material may be at least one selected from the group consisting of an amino acid, a phosphonic acid, a quinoline, a crown ether, a calix-arene, a kryptofix, EDTA, thio-urea, and porphyrin.

In accordance with another aspect, a microfluidic device includes a first reagent chamber to accommodate reagent 1 including an enzyme activated by a target material present in a sample and at least one substrate, reaction of which is catalyzed by the activated enzyme, a second reagent chamber to accommodate reagent 2 including an enzyme activated by the target material, at least one substrate, reaction of which is catalyzed by the activated enzyme, and a capturing material specifically capturing the target material, and a sample inlet through which the sample is injected.

The microfluidic device may further include a first channel to connect the first reagent chamber and the sample inlet and a second channel to connect the second reagent chamber and the sample inlet. The first reagent chamber and the second reagent chamber may not be connected.

The sample may be a biological sample, and the target material may be at least one of electrolyte ions present in the biological sample.

The target material may be at least one of Mg²⁺, Ca²⁺, K⁺, Na⁺, and Cl⁻.

The capturing material may be at least one selected from the group consisting of an amino acid, a phosphonic acid, a quinoline, a crown ether, a calix-arene, a kryptofix, EDTA, thio-urea, and porphyrin.

Further disclosed is a test apparatus to determine a concentration of a target material present in a sample includes a detector to measure first characteristics obtained by a first reaction of the sample and a reagent in the absence of a capturing material specifically capturing the target material specifically capturing the target material and second characteristics obtained by a second reaction of the sample and a reagent in the presence of the capturing material and a control unit to calculate a difference value between the first measured characteristics and the second measured characteristics and to determine the concentration of the target material present in the sample using the calculated difference value.

The control unit may pre-store reference concentration values with regard to various difference values.

The control unit may determine the concentration of the target material present in the sample by referencing a reference concentration value which corresponds to the calculated difference value.

### BRIEF DESCRIPTION OF THE DRAWINGS

These and/or other exemplary embodiments will become apparent and more readily appreciated from the following description of the embodiments, taken in conjunction with the accompanying drawings of which:
FIG. 1 is a schematic view illustrating a conventional process of reducing effects of materials excluding a target material on reaction results by capturing the materials;
FIG. 2 is a flowchart illustrating a sample test method according to an embodiment of the present invention;
FIG. 3 is a schematic view illustrating the concentration of electrolyte ions used in an exemplary embodiment of the sample test method;
FIG. 4 is a schematic view illustrating a process of reaction 1;
FIG. 5 is a schematic view illustrating a process of reaction 2;
FIG. 6 is a schematic view illustrating the concentration of electrolyte ions used in another exemplary embodiment of the sample test method;
FIG. 7 is a graph showing changes in absorbance with respect to time according to concentration of K⁺;
FIG. 8 is a graph showing changes in absorbance with respect to time according to concentration of K⁺ when a certain amount of K⁺ is captured by a capturing material;
FIG. 9 is a graph showing a calibration curve of difference values between absorbance values shown in FIG. 7 and absorbance values shown in FIG. 8;
FIG. 10 is an exterior view of a microfluidic device according to an embodiment;
FIG. 11 is an exploded perspective view illustrating a structure of a test unit of the microfluidic device illustrated in FIG. 10;
FIG. 12 is a top plan view of a microfluidic device according to another embodiment;
FIG. 13 is an exterior view of a test apparatus according to an embodiment;
FIG. 14 is an exterior view of a test apparatus according to another embodiment; and
FIG. 15 is a control block diagram of the test apparatuses of FIGS. 13 and 14.

### DETAILED DESCRIPTION

Reference will now be made in detail to the embodiments of the present invention, examples of which are illustrated in the accompanying drawings, wherein like reference numerals refer to like elements throughout.

Among various methods of measuring the concentration of a target material included in a sample, there is a method of using an enzyme that is activated by a target material and an enzymatic reaction catalyzed by the activated enzyme. The target material means a material, a concentration of which is determined.

In particular, there is an enzymatic method used in an electrolyte test. In the enzymatic method, the concentration of a target material may be determined from the amount of activated enzyme. The kind of an enzyme used, a composition of a reagent, and a reaction mechanism may vary according to the target material. When the target material in a sample is ions such as Mg²⁺, K⁺, and/or Na⁺, for example, an enzymatic reaction using pyruvate kinase, as illustrated below, may be used.

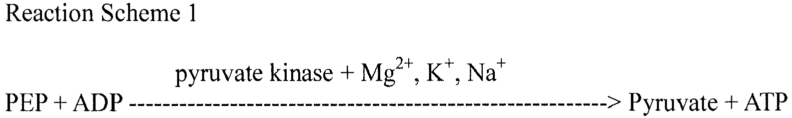

In Reaction Scheme 1, pyruvate kinase is activated by Mg²⁺, K⁺, and Na⁺, and the activated pyruvate kinase acts as a catalyst for reaction between phosphoenolpyruvic acid (PEP), which is a substrate of pyruvate kinase, and adenosine diphosphate (ADP). As a result, pyruvate and adenosine diphosphate (ATP) are produced by reaction between PEP and ADP.

Mg²⁺, K⁺, and Na⁺ are included in a biological sample, in particular a blood sample and thus, when a reagent including PEP, ADP, and pyruvate kinase is added to a blood sample, enzyme reaction according to Reaction Scheme 1 may occur.

ATP, which is the reaction product, is involved in color reaction and thus concentrations of Mg²⁺, K⁺, and Na⁺ may be determined from the measured optical properties of the reaction product. However, in a general electrolyte test, a total concentration of the above-described ions is not needed, but the concentration of at least one of individual Mg²⁺, K⁺, and Na⁺, selected as a target material, is needed. As for a plurality of target materials, concentrations of each of the target materials are needed.

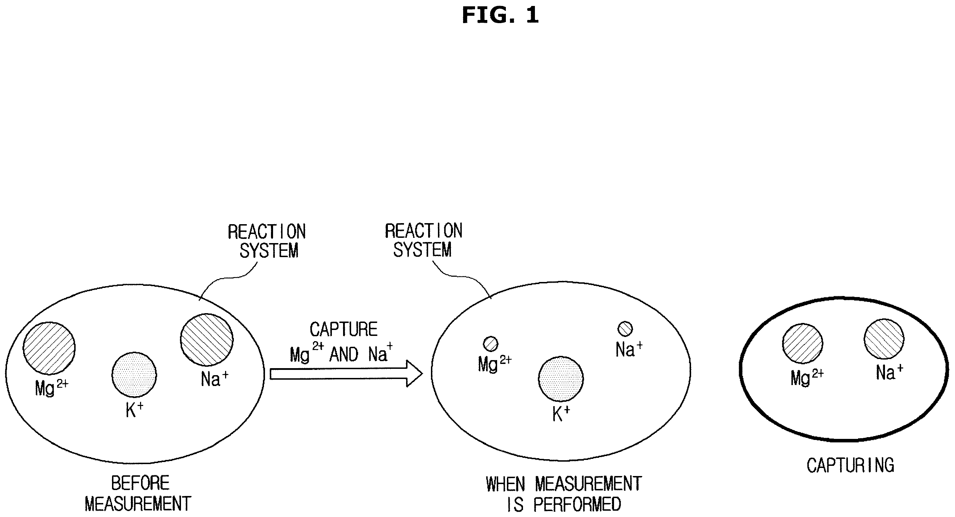

FIG. 1 is a schematic view illustrating a conventional process of reducing interfering effects of non-target materials on reaction results by capturing the non-target materials. In FIG. 1, K⁺ is a target material, and the concentration of the target material is determined using an enzyme reaction (not shown in FIG. 1) according to Reaction Scheme 1. A sample containing the target material may be a blood sample such as serum or plasma.

Referring to FIG. 1, a sample prior to measurement contains K⁺ as a target material, and Mg²⁺, and Na⁺ as non-target materials. As described above, Mg²⁺ and Na⁺ are also involved in the enzyme reaction of Reaction Scheme 1 and thus, conventionally, a method of reducing the amount of non-target Mg²⁺ and Na⁺ by employing an agent which binds to (i.e., capture) Mg²⁺ and Na⁺ in order to reduce an interfering influence of these non-target ions, may be used.

To use this method, a reagent contains an enzyme, reactants (i.e., PEP and ADP) participating in enzyme reaction, and a capturing material that captures Mg²⁺ and Na⁺. However, when the reagent is used as a dry form, a degree of chemical freedom is low and thus it is not easy to control the amounts of a plurality of materials. In addition, a plurality of capturing materials may be required, which may increase the costs of the assays.

Meanwhile, it is possible that other materials than a target material are involved in color reaction. For example, when the enzyme reaction of Reaction Scheme 1 is a main reaction, coloring occurs by consecutive reactions represented by Reaction Schemes 2 to 4 below.

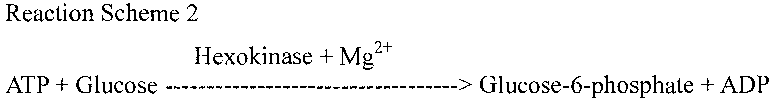

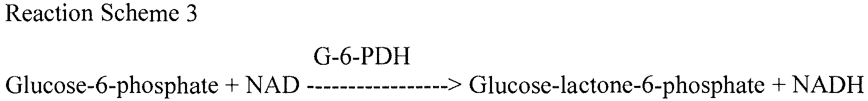

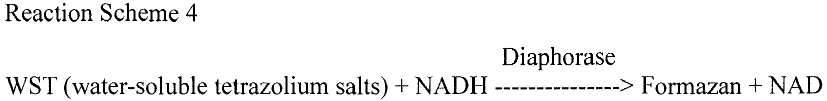

The concentration of the target material is measured from changes in optical characteristics due to the color reaction and thus, when Mg²⁺ is captured, the reaction of Reaction Scheme 2 does not normally occur and thus affects measurement results of the target material.

Thus, an assay method according to an embodiment excludes interfering effects of non-target materials on reaction results by capturing the target material.

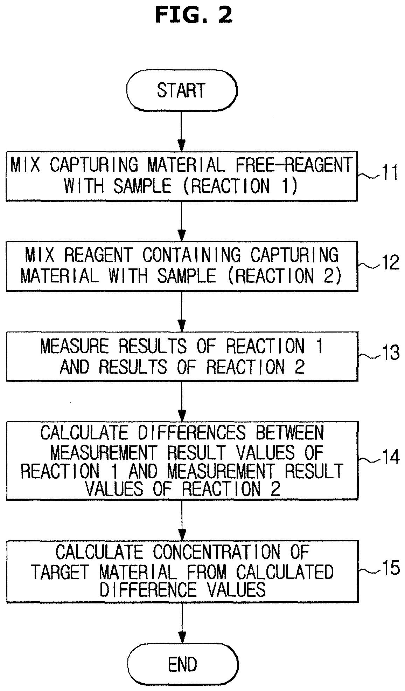

FIG. 2 is a flowchart illustrating a sample test method according to an embodiment.

Referring to FIG. 2, a reagent containing a capturing material that specifically captures a target material is mixed with a sample (operation 12). In the present embodiment, mixing of the reagent with the sample may induce a reaction between the reagent and the sample. Thus, when the reagent containing a capturing material is mixed with the sample, reaction 2 occurs. In this regard, the sample may be a biological sample such as body fluid containing blood, tissue fluid, lymph, urine, and bone marrow fluid or an environmental sample for water quality management, soil management or the like. However, these are merely examples that may be applied to embodiments of the present invention and kind of the sample is not particularly limited so long as it may be measured by an enzymatic method. For convenience of explanation, in embodiments described below, a sample test method by which the concentration of electrolyte ions present in a blood sample is measured by an enzymatic method will be described.

A capturing material-free reagent is mixed with a sample to induce reaction 1 (operation 11). In this regard, the capturing material-free reagent may include the same materials as those of reagent used in reaction 2, except for not including the capturing material.

While FIG. 2 shows reaction 1 (reaction without a capturing material) as being performed prior to reaction 2 (reaction in the presence of a capturing material), the order of reactions 1 and 2 is not limited. That is, reactions 1 and 2 may be simultaneously performed or one reaction is performed prior to the other.

Results of reactions 1 and 2 are measured (operation 13). For example, when color reaction is accompanied by reactions 1 and 2, reaction results are measured after color reaction is completed. Thus, results of reaction 1 and results of reaction 2 may be measured by measuring optical characteristics of each of the two reaction systems.

A difference between a measurement result value of reaction 1 and a measurement result value of reaction 2 is calculated (operation 14). A measurement of characteristics of reactions 1 and 2 may be optically performed, such as fluorescent, luminescent, or the like. When reaction results are measured as optical absorbance or optical density, a difference between an absorbance measurement value of reaction 1 and an absorbance measurement value of reaction 2 is calculated.

Thereafter, the concentration of the target material is determined from the calculated difference value (operation 15). For this operation, information regarding a relationship among the measurement result value of reaction 1 and the measurement result value of reaction 2 at known concentrations of the target material may be pre-stored, and the concentration of the target material may be determined by referencing from the calculated difference value using the relationship.

Hereinafter, an exemplary test method will be described in detail with reference to FIG. 2. In embodiments described below, for convenience of explanation, a capturing material-free reagent is referred to as reagent 1 and a reagent containing a capturing material is referred to as reagent 2.

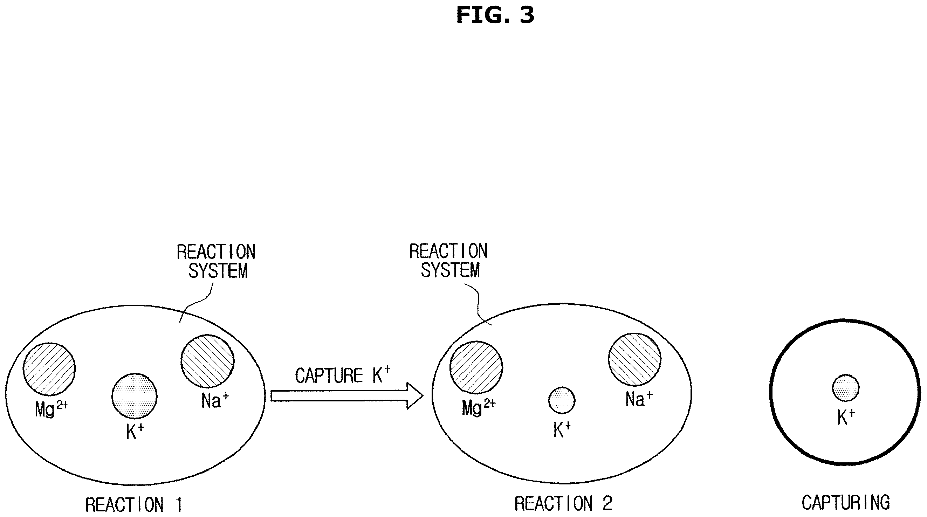

FIG. 3 is a schematic view illustrating the concentration of electrolyte ions used in an example of the sample test method according to an embodiment. In FIG. 3, an enzyme reaction according to Reaction Scheme 1 is denoted as a main reaction and K⁺ is denoted as a target material.

In FIG. 3, only the concentration of electrolyte ions is illustrated. Referring to FIG. 3, in the sample test method according to the embodiment of the present invention, as described above, the concentration of the target material is measured using the two reactions (i.e., reactions 1 and 2). In a reaction system in which reaction 1 occurs, Mg²⁺, K⁺, and Na⁺, which are electrolyte ions, are present in the sample. That is, capturing of target electrolyte ions is not performed.

Meanwhile, in a reaction system in which reaction 2 occurs, a smaller amount of K⁺ is present than in reaction 1. That is, a capturing material that captures K⁺ is included in a reagent added to a sample so that a certain amount of K⁺ does not participate in an enzymatic reaction. A reduction in concentration of K⁺ is proportionate to the concentration of the capturing material, and the capturing material that captures K⁺ is a material that selectively captures K⁺ among Mg²⁺, K⁺, and Na⁺. Thus, the capturing material that captures K⁺ does not affect the concentrations of Mg²⁺ and Na⁺ in the sample.

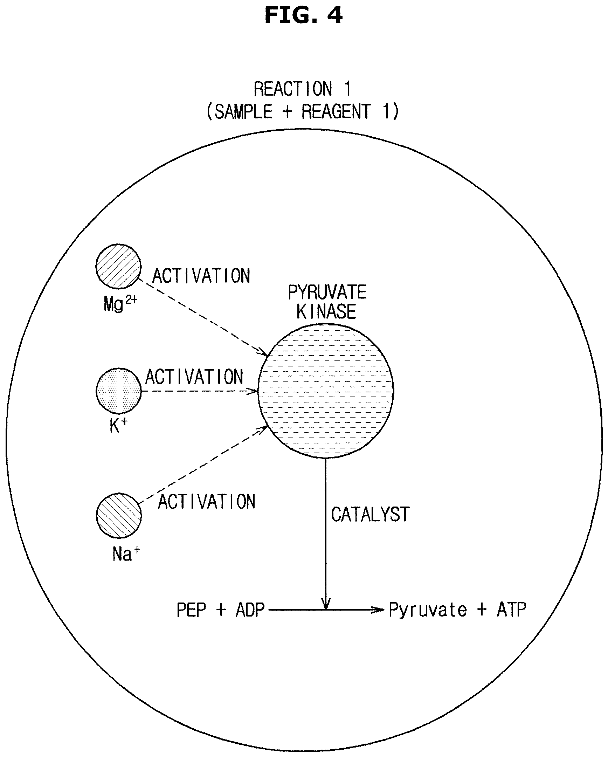

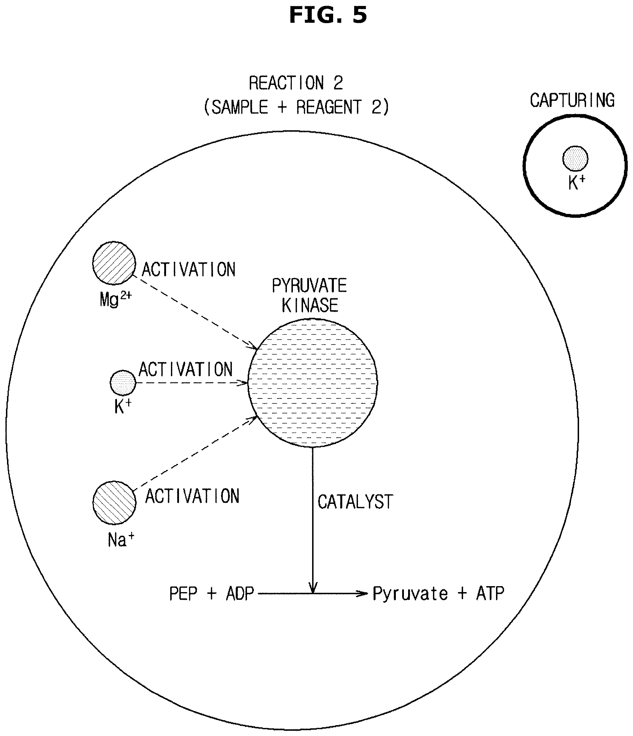

FIG. 4 is a schematic view illustrating a process of reaction 1. FIG. 5 is a schematic view illustrating a process of reaction 2.

Referring to FIG. 4, in reaction 1, reagent 1 is added to a sample containing electrolyte ions. In this regard, reagent 1 is a reagent including substrates (PEP and ADP) and an enzyme (pyruvate kinase) and excluding a capturing material.

In reaction 1, electrolyte ions present in the sample, i.e., Mg²⁺, K⁺, and Na⁺, are all involved in activation of pyruvate kinase as an enzyme, and pyruvate kinase activated by Mg²⁺, K⁺, and Na⁺ catalyzes a reaction between PEP and ADP. Thus, a measurement value of reaction 1 represents a total concentration of Mg²⁺, K⁺, and Na⁺.

Referring to FIG. 5, in reaction 2, reagent 2 is added to the sample containing electrolyte ions. In this regard, reagent 2 is a reagent including substrates (PEP and ADP), an enzyme (pyruvate kinase), and a capturing material.

In reaction 2, electrolyte ions present in the sample, i.e., Mg²⁺, K⁺, and Na⁺, are all involved in activation of pyruvate kinase as an enzyme, while the capturing material captures a certain amount of K⁺, which is a target material, and thus the concentration of K⁺ involved in activation of pyruvate kinase is lower than that in reaction 1. As a capturing material which specifically binds to K⁺, but not to Mg²⁺ and Na⁺ may be exemplified by cryptands such as KRYPTOFIX® 222 or Calix(8)arene, among others.

Changes in concentration of K⁺ affect the concentration of the activated pyruvate kinase in a reaction mixture and, consequently, the measurement value of reaction 2 is different from the measurement value of reaction 1, wherein the difference is attributed to the changes in the concentration of K⁺.

Thus, the concentration of K⁺ may be calculated from the difference between the measurement value of reaction 1 and the measurement value of reaction 2.

As another exemplary test method, a method for determining the concentration of Cl⁻ will be explained. Measurement of the concentration of Cl⁻ using amylase may be performed by an enzymatic reaction according to Reaction Scheme 5 below:

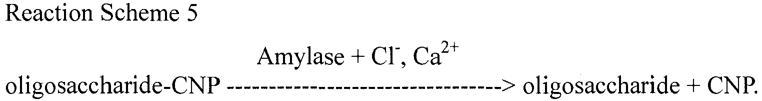

According to Reaction Scheme 5, amylase is activated by Cl⁻ and Ca²⁺ present in a blood sample, and the activated amylase catalyzes the cleavage of oligosaccharide- 2-chloro-p-nitrophenol (CNP) complex, i.e., oligosaccharide-CNP, to produce oligosaccharide and CNP.

CNP acts as a color coupler and thus is used to measure optical characteristics of reaction products and, accordingly, the concentrations of Cl⁻ and Ca²⁺ may be calculated from the absorbance of CNP. As described above, the concentration calculated from the measurement value of optical characteristics is a total concentration of Cl⁻ and Ca²⁺ and thus to calculate individual concentration of a target ion, a certain amount of the target ion may be captured according to the substantially similar method described above by employing an appropriate capturing material.

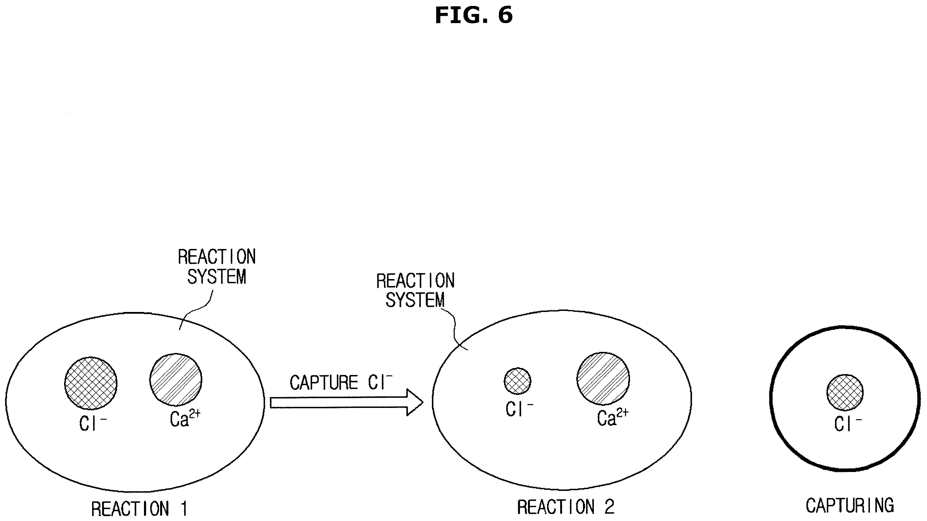

FIG. 6 is a schematic view illustrating the concentration of electrolyte ions used in another exemplary test method. In FIG. 6, the enzymatic reaction according to Reaction Scheme 5 is denoted as a main reaction and Cl⁻ is denoted as a target material.

In FIG. 6, only the concentration of electrolyte ions is illustrated. Referring to FIG. 6, in the present embodiment, as described above, the concentration of the target material is measured using the two reactions (reactions 1 and 2). In a reaction system in which reaction 1 occurs, electrolyte ions present in the sample, i.e., Cl⁻ and Ca²⁺, are present. That is, capturing of the electrolyte ion as a target material is not performed.

Meanwhile, in a reaction system in which reaction 2 occurs, a smaller amount of Cl⁻ is present than in reaction 1. That is, the existence of a capturing material that specifically captures Cl⁻ reduces the amount of Cl⁻ in the enzymatic reaction. A reduction in the concentration of Cl⁻ is proportionate to the concentration of the capturing material that selectively captures Cl⁻ out of a sample containing both Ca²⁺ and Cl⁻. Thus, the capturing material that captures Cl⁻ does not affect the concentration of Ca²⁺ in the sample.

In reaction 1, electrolyte ions present in the sample, i.e., Ca²⁺ and Cl⁻, are all involved in activation of amylase as an enzyme, and amylase activated by Ca²⁺ and Cl⁻ acts as a catalyst in decomposition of oligosaccharide-CNP. Thus, an optical characteristic measurement value of reaction 1 represents a total concentration of Ca²⁺ and Cl⁻.

In reaction 2, electrolyte ions present in the sample, i.e., Ca²⁺ and Cl⁻, are all involved in activation of amylase as an enzyme, while the capturing material selectively captures a certain amount of Cl⁻, which is a target material, and thus, the concentration of Cl⁻ involved in activation of amylase is lower than that in reaction 1. As a capturing material which specifically binds Cl⁻, but not Ca²⁺ may include.

Changes in concentration of Cl⁻ affect the concentration of activated amylase and, consequently, a difference occurs between the measurement value of reaction 2 and the measurement value of reaction 1 and this is only in accordance with changes in concentration of Cl⁻.

Thus, the concentration of Cl⁻ may be calculated from the difference between the measurement value of reaction 1 and the measurement value of reaction 2.

Various materials may be used as the capturing material that captures an electrolyte ion. For example, a chelating agent may be used as the capturing material. Appropriate capturing material may be chosen depending on the target ion. For example, N, N, N', N'-tetrakis (2-pyridylmethyl)-ethylenedidiamine (TPEN) shows strong affinities for Zn²⁺, Fe²⁺, and Mn²⁺, while KRYPTOFIX® 222 or Calix(8)arene shows strong affinity for K⁺. In Table 1 below, several chelating agents that may be used as the capturing material are shown.

**[Table 1]**

| Target material | Capturing material 1 | Capturing material 2 |
|---|---|---|
| K⁺ | 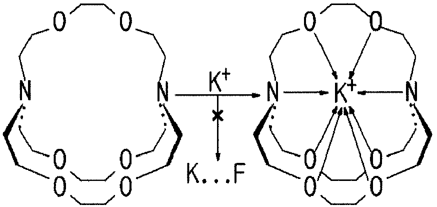 | 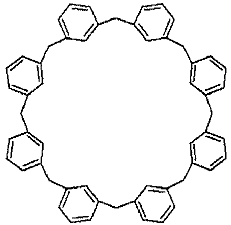 |
| | Kryptofix 222 | Calix(8) arene |
| Na⁺ | 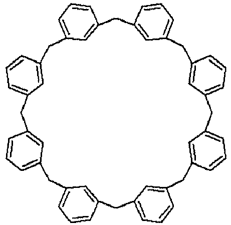 | 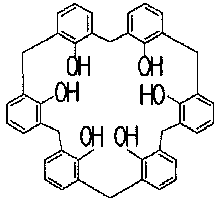 |
| | Kryptofix 221 | Calix(6) arene |
| Mg² | 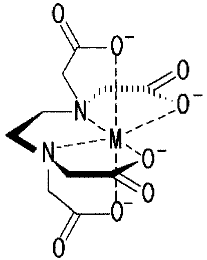 | 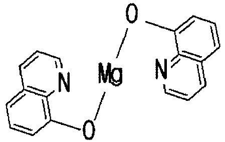 |
| | EDTA | 8-Quinolinol |

In addition, examples of the capturing material include, but are not limited to, amino carboxylic acids such as ethylenediaminetetraacetic acid (EDTA), nitrilotriacetic acid (NTA), diethylene triamine pentaacetic acid (DTPA), N-(2-Hydroxyethyl)ethylenediamine-N,N',N'-triacetic acid (HEDTA), triethylenetetramine-N,N,N',N",N"',N"'-hexaacetic Acid (TTHA), propylene diamine tetra acetic acid (PDTA), 1,3-diamino-6-hydroxypropane tetraacetic acid (DPTA-OH), N-(2-hydroxyethyl)iminodiacetic acid (HIDA), dihydroxyethylglycine (DHEG), glycoletherdiaminetetraacetic acid (GEDTA), 1,4,7,10-tetraazacyclododecane-1,4,7,10-tetraacetic acid (DOTA), dicarboxymethyl Glutamic Acid (CMGA), 1,2-bis(2-aminophenoxy)ethane-N,N,N',N'-tetraacetic acid (BAPTA), Bicine, trans-1,2-cyclohexanediaminetetraacetic acid monohydrate (CyDTA), ethylene glycol tetraacetic acid (EGTA), iminodiacetic acid (IDA), nitrilotriacetic acid (NTA), Triethylenetetramine-N,N,N',N",N"',N"'-hexaacetic Acid (TTHA), and (S,S)-ethylenediamine disuccinic acid (EDDS); phosphonic acids such as 1-hydroxy ethidene -1,1-diphosphonic acid (HEDP), nitrilotris(methylene)phosphonic acid (NTMP), phosphonobutane tricarboxylic Acid (PBTC), ethylene diamine tetra(methylene phosphonic acid) (EDTMP), and nitrilotrimethylphosphonic acid (NTP); quinolines such as 4-hydroxy quinoline, 8-phenyl quinoline, and quinolone; crown ethers such as 12-crown-4, 15-crown-5, 18-crown-6, di-benzo-18-crown-6, and di-aza-18-crown-6; and calix-arenes such as Calix(8) arene, Calix(6) arene, and Calix(4) arene.

In addition, N, N, N', N'-tetrakis (2-pyridylmethyl)-ethylenedidiamine (TPEN), porphyrin, phenanthroline, iminodiacetic acid, etidronic acid, phthalocyanine, salicylic acid, urea, thio-urea, or the like may be used as the capturing material.

In Table 2 below, structures of some of the above-listed capturing materials are shown.

**[Table 2]**

| Capturing material | Structure | | | | |
|---|---|---|---|---|---|
| Quinoline | 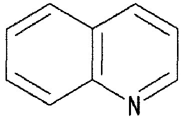 | | | | |
| Grown ether | 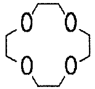 | 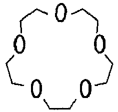 | 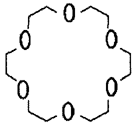 | 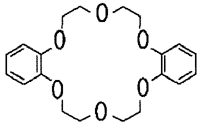 | 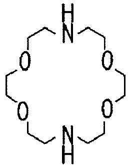 |
| | 12-crown-4, | 15-crown-5, | 18-crown-6, | di-benzo-18-crown-6, | di-aza-18-crown-6 |
| Porphyrin | 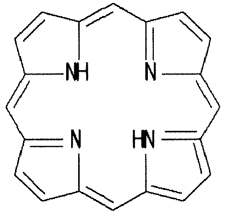 | | | | |

The above-listed exemplary capturing materials may capture a target material using structural properties thereof or by electrical attraction force, and an appropriate capturing material may be chosen according to kind of target material to be captured.

For example, in urea having formula CO(NH₂)₂ and thio-urea having formula CS(NH₂)₂, within the amine (-NH₂) groups, hydrogen (H) electrons are attracted towards nitrogen (N) having strong electronegativity and thus hydrogen is positively charged, and Cl⁻ having negative charge approaches the positively charged hydrogen, thereby forming a hydrogen bond. That is, Cl⁻ ions are captured by this hydrogen bond.

However, the above-described capturing materials are merely examples that may be applied to embodiments of the present invention and embodiments of the present invention are not limited to the above examples. Any capturing material capable of selectively capturing the target material may be used as the capturing material used in embodiments of the present invention.

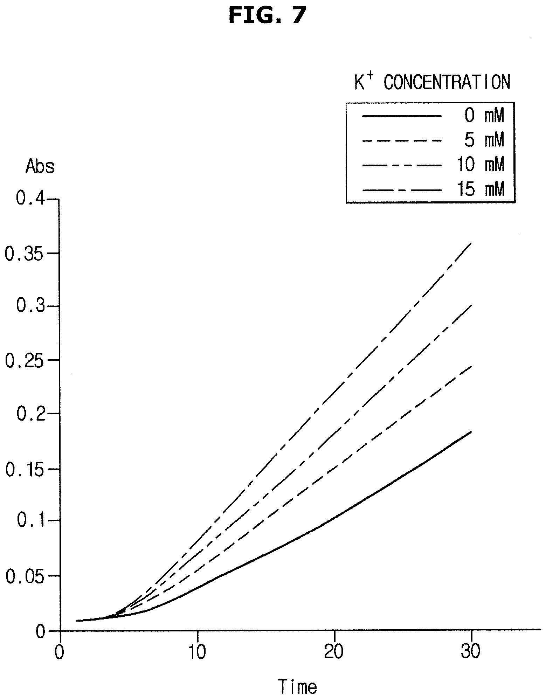

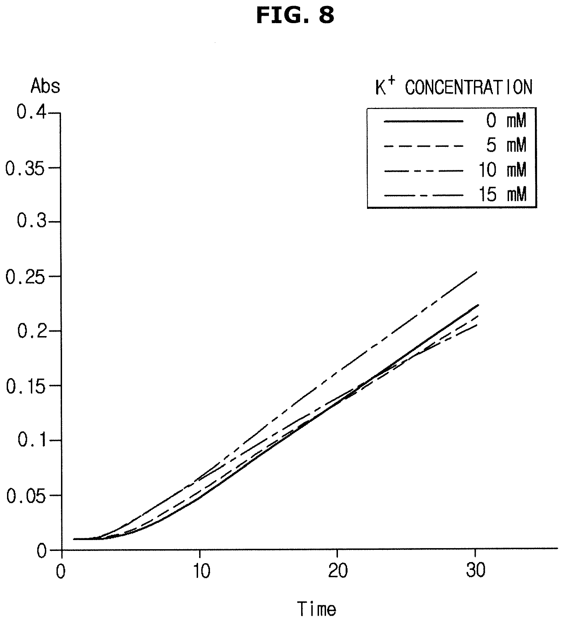

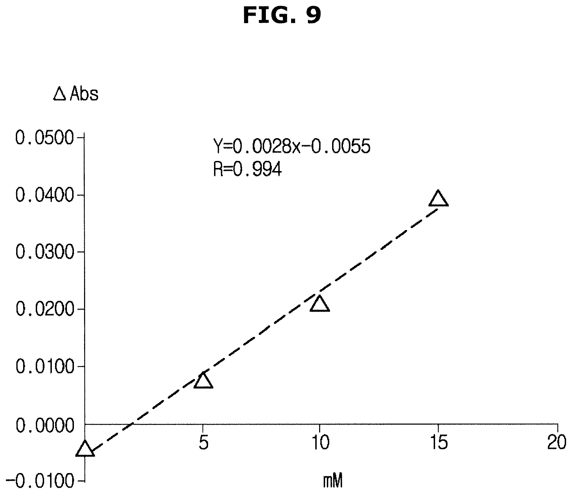

FIG. 7 is a graph showing changes in absorbance with respect to time according to various known concentrations (i.e., 0, 5, 10, and 15 mM) of K⁺. FIG. 8 is a graph showing changes in absorbance with respect to time according to various known same concentrations (i.e., 0, 5, 10, and 15 mM) of K⁺ as those in the test of FIG. 7, except the reaction was carried out in the presence of a capturing material. FIG. 9 is a graph showing a calibration curve of difference values between absorbance values shown in FIG. 7 and absorbance values shown in FIG. 8.

FIGS. 7 to 9 illustrate experimental results. In the present experimental examples, K⁺ was used as a target material and the enzymatic reaction according to Reaction Scheme 1 was used. KRYPTOFIX® 222 was used as a capturing material and the concentration of KRYPTOFIX® 222 was 10mM. Measurement wavelengths were 630 nm and 810 nm. Main wavelength was 630nm and sub wavelength was 810nm. Accordingly, an absorbance value at 810nm was subtracted from an absorbance value at 630nm.

In the graphs shown in FIGS. 7 and 8, the horizontal axis denotes time (second) and the vertical axis ("Abs") denotes absorbance. The graph of FIG. 7 shows reference measurement results of reaction 1 when the concentration of K⁺ is 0 mM, 5 mM, 10 mM, and 15 mM, that is, when K⁺ was not captured. The graph of FIG. 8 shows measurement results of reaction 2 when the concentration of K⁺ is 0 mM, 5 mM, 10 mM, and 15 mM, that is, when a certain amount of K⁺ was captured. In this regard, a same amount of KRYPTOFIX® 222 was used in each tests regardless of the concentrations of K⁺ and the amount of K⁺ captured was constant.

In addition, a calibration curve obtained using difference values obtained by subtracting absorbance values illustrated in FIG. 8 from absorbance values illustrated in FIG. 7 is illustrated in FIG. 9. In FIG. 9, the horizontal axis denotes the concentration (mM) of K⁺ and the vertical axis (Δ Abs) denotes difference values between the measured absorbance values of FIG. 7 and FIG. 8 at each of the concentration of K⁺. As illustrated in FIG. 9, the calibration curve represents good linearity and thus may be represented by the following equation: y= 0.0028x-0.0055, and correlation coefficient R is 0.994.

As described above, information regarding a relationship between the differences between measured values of reactions 1 and 2 and the concentrations of the target material may be pre-stored and the concentration of the target material in a test sample may be calculated from the calculated difference values using this relationship when the difference values of the measured values of reaction 1 and reaction 2 are calculated.

In the previous embodiment, the concentration of the target material is calculated by measuring optical properties of reaction products, but embodiments of the present invention are not limited thereto. That is, electrical properties of reaction products may be used to calculate the concentration of the target material. For example, the electrical properties of reaction products may be measured by exposing an electrode sensor to a chamber in which reaction between reagent 1 and a sample occurs and a chamber in which reaction between reagent 2 and a sample occurs. In addition, the concentration of the target material may be calculated using an output signal of the electrode sensor.

A reagent kit according to an embodiment may include reagents 1 and 2 used in the sample test method described above. That is, the reagent kit according to the embodiment may include two kinds of reagent compositions and these reagent compositions may have various compositions according to kind of a target material, a concentration of which is to be measured.

In particular, the reagent kit according to the embodiment may include reagent 1 which contains an enzyme that is activated by a target material, at least one substrate of the enzyme, and reagent 2 which contains an enzyme that is activated by a target material, at least one substrate of the enzyme, and a capturing material that captures a target material. Reagents 1 and 2 may include other agents or substances which may be required or preferred to facilitate a reaction by the enzyme upon the substrate. For example, the reagents may include commonly known buffers. The capturing material selectively or specifically captures the target material. Herein, the term "selectively" or "specifically" is used to mean the capturing material shows significantly higher affinity toward the target material and do not or substantially not bind to non-target materials.

In this regard, the target material may be an electrolyte ion present in a biological sample, in particular a blood sample, and the enzyme activated by the target material may vary according to kind of the target material. For example, the target material may be an electrolyte ion such as K⁺, Mg²⁺. Cl⁻, Na⁺, Ca²⁺, or the like, and the enzyme may pyruvate kinase, amylase, α-amylase, or the like.

The substrate of an enzyme, i.e., substance that is cleaved or changed by an action of the enzyme, may vary according to kind of the enzyme. For example, when pyruvate kinase is used as an enzyme, PEP and APD may be used as substrates. When amylase is sued as an enzyme, oligosaccharide-CNP may be used as a substrate. When α-amylase is used as an enzyme, 2-chloro-4-nitrophenyl-α-D-maltotrioside (CNPG3) may be used as a substrate. However, the above-described materials are merely examples that may be applied to embodiments of the present invention and embodiments of the present invention are not limited to the above examples.

The capturing material may vary according to kind of the target material. The capturing material may be one of the chelating agents shown in Table 1 above. In another embodiment, an amino carboxylic acid such as EDTA, NTA, DTPA, HEDTA, TTHA, PDTA, DPTA-OH, HIDA, DHEG, GEDTA, DOTA, CMGA, BAPTA, Bicine, CyDTA, GEDTA (EGTA), IDA, NTA, TTHA, EDDS, or the like, a phosphonic acid such as HEDP, NTMP, PBTC, EDTMP, NTPO, or the like, a quinoline such as 4-hydroxy quinoline, 8-phenyl quinoline, quinolone, or the like, a crown ether such as 12-crown-4, 15-crown-5, 18-crown-6, di-benzo-18-crown-6, di-aza-18-crown-6, or the like, or a calix-arene such as Calix(8) arene, Calix(6) arene, Calix(4) arene, or the like may be used as the capturing material.

In addition, TPEN, porphyrin, phenanthroline, iminodiacetic acid, etidronic acid, phthalocyanine, salicylic acid, urea, thio-urea, or the like may be used as the capturing material.

However, the above-described capturing materials are merely examples of capturing materials that may be used in embodiments of the present invention and capturing materials included in the reagent set are not limited to the above examples. Any capturing material capable of selectively capturing the target material may be used as the capturing material used in embodiments of the present invention.

Meanwhile, a composition ratio of materials constituting the reagent composition may be appropriately adjusted according to various test conditions. For example, a reagent composition may be prepared by adding a certain amount of capturing material to the composition of reagent 1, which is an existing reagent used to measure the concentration of electrolyte ions, and the concentration of capturing material added may be made constant according to the concentration of capturing material used to draw the calibration curve.

Hereinafter, a microfluidic device according to an embodiment of the present invention will be described. The microfluidic device according to the embodiment of the present invention may be used to perform the sample test method according to the aforementioned embodiment.

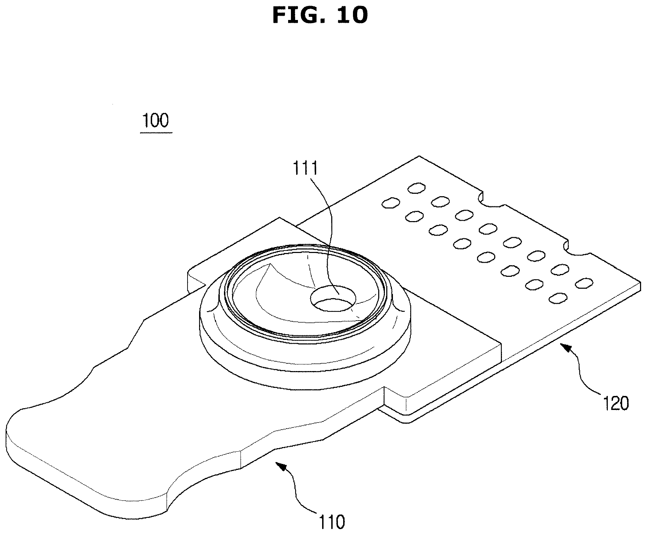

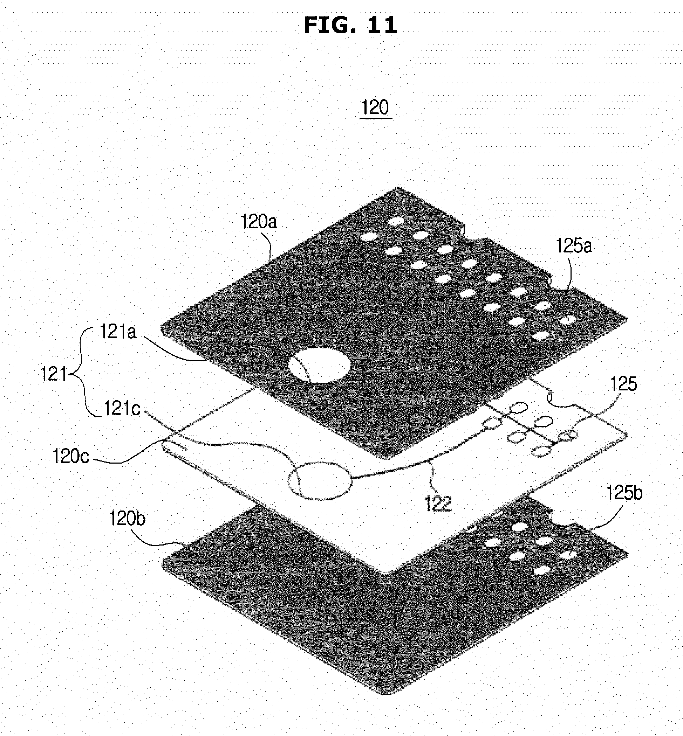

FIG. 10 is an exterior view of a microfluidic device 100 according to an embodiment of the present invention. FIG. 11 is an exploded perspective view illustrating a structure of a test unit 120 of the microfluidic device 100 illustrated in FIG. 10.

Referring to FIG. 10, the microfluidic device 100 according to the present embodiment may be an analytical cartridge including a housing 110 and the test unit 120 in which reaction between a sample and a reagent occurs.

The housing 110 supports the test unit 120 and allows a user to grab the microfluidic device 100. The housing 110 may be formed of a material that is easy to mold and is chemically and biologically inert.

Examples of materials for forming the housing 110 include acryls such as polymethylmethacrylate (PMMA), polysiloxanes such as polydimethylsiloxane (PDMS), polycarbonate (PC), polyethylenes such as linear low density polyethylene (LLDPE), low density polyethylene (LDPE), medium density polyethylene (MDPE), and high density polyethylene (HDPE), plastic materials such as polyvinyl alcohols, very low density polyethylene (VLDPE), polypropylene (PP), acrylonitrile butadiene styrene (ABS), and cycloolefin copolymer (COC), glass, mica, silica, and a semiconductor wafer. However, materials of the housing 110 are not limited to the above examples.

The housing 110 includes a sample supply unit 111 to supply a sample. As described above with regard to the embodiment of the sample test method, the sample supplied to the microfluidic device 100 may be a biological sample such as body fluid containing blood, tissue fluid, lymph, urine, and bone marrow fluid or an environmental sample for water quality management, soil management or the like, and the target material, a concentration of which is to be measured, may be an electrolyte ion present in the sample.

The test unit 120 may be adhered to a lower portion of the sample supply unit 111 of the housing 110 or coupled with the housing 110 by being engaged with predetermined grooves formed in the housing 110.

The sample supplied via the sample supply unit 111 is introduced into the test unit 120 via a sample inlet 121 arranged at the test unit 120. Although not shown, a filter may be disposed between the sample supply unit 111 and the sample inlet 121 to filter the sample supplied via the sample supply unit 111. The filter may be a porous polymer membrane formed of polycarbonate (PC), polyethersulfone (PES), polyethylene (PE), polysulfone (PS), polyarylsulfone (PASF), or the like.

For example, in a case in which a blood sample is supplied, while blood passes through the filter, blood cells remain and only blood plasma or serum may be introduced into the test unit 120.

Referring to FIG. 11, the test unit 120 may have a structure in which three plates 120a, 120b and 120c are adhered to one another. The three plates 120a, 120b and 120c may include an upper plate 120a, a lower plate 120b, and a middle plate 120c. The upper and lower plates 120a and 120b are printed with light shielding ink and thus may protect a sample being moved into a reagent chamber 125 from external light.

The upper and lower plates 120a and 120b may be formed of a polymer film. The polymer film used to form the upper and lower plates 120a and 120b may be one selected from among a VLDPE film, a LLDPE film, a LDPE film, a MDPE film, a HDPE film, a PP film, a polyvinyl chloride (PVC) film, a polyvinyl alcohol (PVA) film, a polystyrene (PS) film, and a polyethylene terephthalate (PET) film.

The middle plate 120c of the test unit 120 may be a porous sheet formed of cellulose or the like and thus may serve as a vent. In this regard, the porous sheet may be formed of a hydrophobic material or may be hydrophobically treated so as not to affect movement of the sample.

The test unit 120 includes the sample inlet 121, a channel 122 through which the introduced sample moves, and the reagent chambers 125 in which reaction between the sample and the reagent occurs. As illustrated in FIG. 11, when the test unit 120 has a three layered structure, the upper plate 120a is provided with an upper plate hole 121a constituting the sample inlet 121 and portions 125a corresponding to the reagent chambers 125 may be transparent.

In addition, portions 125b of the lower plate 120b, corresponding to the reagent chambers 125, may be transparently treated. In this regard, the portions 125a and 125b corresponding to the reagent chambers 125 are transparent so as to allow measurement of optical properties by reaction occurring in the reagent chambers 125.

The middle plate 120c is also provided with a middle plate hole 121c constituting the sample inlet 121. When the upper, middle and lower plates 120a, 120c and 120b are adhered to one another, the upper and middle holes 121a and 121c overlap each other to form the sample inlet 121 of the test unit 120.

The reagent chambers 125 are formed at a region of the middle plate 120c, opposite to the middle hole 121c. For example, the reagent chambers 125 may be formed by removing regions of the middle plate 120c corresponding to the reagent chambers 125 into a certain shape such as a circle, a tetragon, or the like and adhering the upper, middle and lower plates 120a, 120b and 120c.

In addition, the channel 122 having a width of 1 µm to 500 µm is formed at the middle plate 120c and thus the sample introduced via the sample inlet 121 may be moved to the reagent chambers 125 by pressure of the channel 122. However, the width of the channel 122 is merely an example that may be applied to the microfluidic device 100 and embodiments of the present invention are not limited thereto.

The reagent chambers 125 may include pre-loaded reagents used to detect a target material. For example, one of the reagent samples 125 may include reagent 1 and another thereof may include reagent 2. A detailed description of reagents 1 and 2 has already been provided above.

As an example of pre-loading reagents, reagents in liquid state may be respectively applied to the portions 125a of the upper plate 120a corresponding to the reagent chambers 125 or the portions 125b of the lower plate 120b corresponding to the reagent chambers 125, followed by drying, preferably freeze-drying. Or, the reagents may be accommodated in the form of a dry reagent through adhesion of the upper, lower and middle plates 120a, 120b and 120c.

When a sample is supplied to the sample supply unit 111 of the microfluidic device 100, the supplied sample is introduced into the test unit 120 via the sample inlet 121 and the introduced sample moves to each reagent chamber 125 along the channel 122.

The sample is mixed with each of reagents 1 and 2 in the respective reagent chambers 125 to induce reactions 1 and 2. Thus, optical characteristics of a reagent chamber in which reaction 1 occurs and optical characteristics of a reagent chamber in which reaction 2 occurs are measured and difference values therebetween are calculated, and the concentration of target material present in the sample may be calculated using a reference relationship between difference values and the concentration of target material and the calculated difference values.

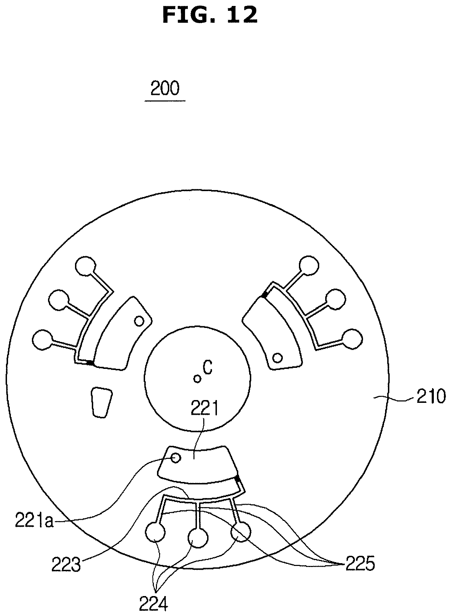

FIG. 12 is a top plan view of a microfluidic device 200 according to another embodiment of the present invention.

Referring to FIG. 12, the microfluidic device 200 according to the embodiment of the present invention may include a rotatable platform 210 and microfluidic structures formed at the platform 210.

The microfluidic structures include a plurality of chambers to accommodate a sample or a reagent and channels to connect the chambers. The microfluidic structures are formed inside the microfluidic device 200. In the present embodiment, however, assuming that the microfluidic device 200 is made of a transparent material, as illustrated in FIG. 12, when viewed from a top side, the microfluidic structures formed in the microfluidic device 200 may be visible.

The platform 210 may be made of a material that is easy to mold, a surface of which is biologically inert. For example, the platform 210 may be made of various materials, for example, plastic materials such as an acryl (PMMA), PDMS, PC, PP, PVA, PE, and the like, glass, mica, silica, a silicon wafer, and the like.

However, embodiments of the present invention are not limited to the above examples and any material that has chemical and biological stability and mechanical processability may be used as the material for forming the platform 210. In addition, when test results of the microfluidic device 200 are optically analyzed, the platform 210 may further have optical transparency.

The microfluidic device 200 may move materials in the microfluidic structures using centrifugal force by rotation. Although FIG. 12 illustrates the platform 210 as a circular plate-shaped disk, the platform 210 applied to embodiments of the present invention may have a completely circular plate shape, a sector shape, or the like and may also have a polygonal shape so long as the platform 210 is rotatable.

The microfluidic structures used herein do not denote particularly shaped structures, but comprehensively denote structures such as chambers or channels formed on the platform 210 and, as desired, may also comprehensively denote materials that implement specific functions. The microfluidic structures may serve different functions according to disposition characteristics or kind of accommodated material.

The platform 210 includes a sample inlet 221a, a sample supply chamber 221 to accommodate a sample injected via the sample inlet 221a and supply the sample to another chamber, reagent chambers 224 in which reaction between a reagent and the sample occurs, and a distribution channel 223 to distribute the sample accommodated in the sample supply chamber 221 to the reagent chambers 224. In addition, although not shown, when blood is used as the sample, the microfluidic device 200 may further include a microfluidic structure for centrifugation of blood.

As illustrated in FIG. 12, when the reagent chambers 224 are formed, a plurality of branch channels 225 may be branched from the distribution channel 223 to connect the distribution channel 223 and each of the reagent chambers 224.

The reagent chambers 224 may pre-accommodate reagents used to detect a target material. For example, reagent 1 may be accommodated in one of the reagent chambers 224 and reagent 2 may be accommodated in another thereof. A detailed description of reagents 1 and 2 has already been given above.

The platform 210 may be formed of multiple layers of plates. For example, when the platform 210 is formed of two plates, i.e., upper and lower plates, a space to accommodate a fluid in the platform 210 and a passage through which the fluid flows may be provided by forming intaglio structures corresponding to the microfluidic structures, such as chambers, channels, and the like, at a contact surface between the upper and lower plates and adhering the two plates. Adhesion between the upper and lower plates may be performed using an adhesive or a double-sided adhesive tape or various methods such as ultrasonic welding, laser welding, and the like.

Thus, to accommodate reagents in the reagent chambers 224, reagents 1 and 2 may be accommodated in portions of the upper or lower plate of the platform 210 at which the intaglio structures corresponding to the reagent chambers 224 are formed and the upper and lower plates may be adhered. Before adhering the upper and lower plates, accommodated reagents may be dried.

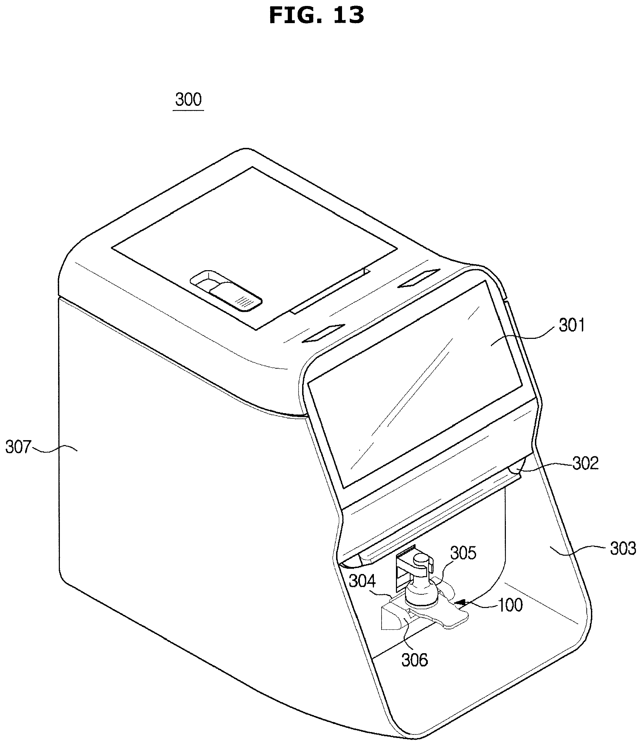

FIG. 13 is an exterior view of a test apparatus 300 according to an embodiment. The test apparatus 300 according to the present embodiment may be used to test the microfluidic device 100 according to the embodiment of the present invention.

The test apparatus 300 is a small-scale automated device for use in testing various kinds of samples such as an environmental sample, a bio-sample, a food sample, and the like. In particular, when the test apparatus 300 is used for in vitro diagnosis by which a biological sample collected from human bodies is tested, in vitro diagnosis may be promptly performed by a user such as a patient, a doctor, a nurse, a medical laboratory technician, or the like in places such as the home, workplace, an outpatient examination room, a hospital room, an emergency room, an operating room and an intensive care unit, in addition to a test room. Referring to FIG. 13, the test apparatus 300 includes an installation unit 303, which is a space in which the microfluidic device 100 is installed, and the microfluidic device 100 may be installed in the test apparatus 300 after opening a door 302 of the installation unit 303 through upward sliding. In particular, the test unit 120 (see FIGs. 10-11) of the microfluidic device 100 may be inserted into a predetermined insertion groove 304 arranged at the installation unit 303.

The test unit 120 may be inserted into a main body 307 and the housing 110 (see FIG. 10) may be exposed to the outside of the test apparatus 300 and supported by a support body 306. In addition, when a pressurization unit 305 pressurizes the sample supply unit 111 (see FIG. 10), introduction of a sample into the test unit 120 may be accelerated.

After introduction into the test unit 120, the sample moves through the channel 122 and reaches one of the reagent chambers 125 in which reagent 1 is accommodated and another thereof in which reagent 2 is accommodated, and an enzymatic reaction occurs in each reagent chamber 125 by an electrolyte ion included in the sample.

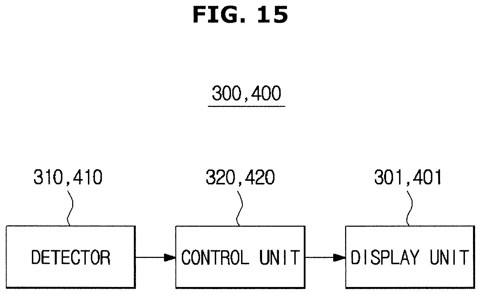

When installation of the microfluidic device 100 is completed, the door 302 is closed and a test starts. Although not shown, the main body 307 includes a detector 310 (see FIG. 15) to measure optical characteristics or electrical characteristics that vary according to concentration of the target material. For example, when measuring optical characteristics, the detector 310 irradiates the reagent chamber 125 in which reagent 1 is accommodated and the reagent chamber 125 in which reagent 2 is accommodated with light and detects light that is reflected or transmitted from each reagent chamber 125.

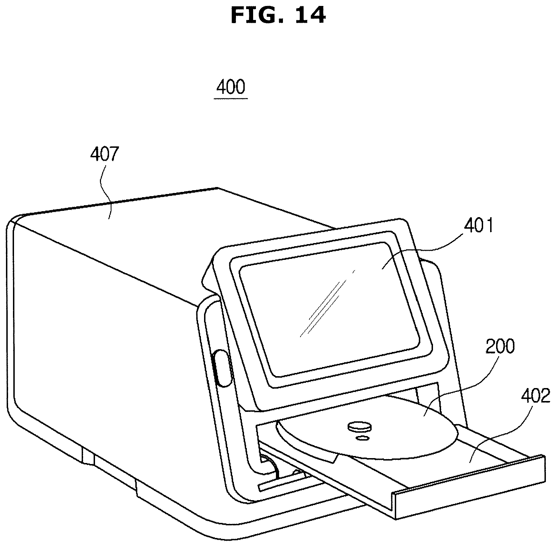

FIG. 14 is an exterior view of a test apparatus 400 according to another embodiment. The test apparatus 400 according to the present embodiment is used to test the microfluidic device 200 according to the above-described embodiment.

Referring to FIG. 14, the microfluidic device 200 is seated on a tray 402 of the test apparatus 400. The seated microfluidic device 200 is inserted into a main body 407 of the test apparatus 400 together with the tray 402. When the microfluidic device 200 is inserted, the test apparatus 400 rotates the microfluidic device 200 according to a sequence determined in accordance with kind of inserted microfluidic device or kind of a test, and a sample injected into the sample supply chamber 221 is moved to the reagent chambers 225 by centrifugal force.

When reaction in the reagent chambers 225 is completed, optical characteristics or electrical characteristics of each of a reaction product of reaction 1 occurring in the reagent chamber 225 in which reagent 1 is accommodated and a reaction product of reaction 2 occurring in the reagent chamber 225 in which reagent 2 is accommodated are measured using a detector 410 (see FIG. 15) arranged in the test apparatus 400.

FIG. 15 is a control block diagram of the test apparatus 300 of FIG. 13 and the test apparatus 400 of FIG. 14.

Hereinafter, sample test operations of the test apparatus 300 or 400 will be described with reference to FIG. 15. Although there are slight differences in exterior views or operations until reaction occurs between the test apparatus 300 of FIG. 13 and the test apparatus 400 of FIG. 14, operations after measurement of optical characteristics of the test apparatuses 300 and 400 are identical as follows.

The test apparatus 300 or 400 may measure optical characteristics or electrical characteristics, such as absorbance, transmittance, a degree of luminescence, and reflectance, from an output signal of the detector 310 or 410. As described above, the concentration of target material present in a sample may be determined using the measured optical characteristics or electrical characteristics of the reagent chamber 125 or 224 in which reagent 1 is accommodated and the reagent chamber 125 or 224 in which reagent 2 is accommodated.

Hereinafter, for convenience of explanation, the measured optical characteristics of the reagent chamber 125 or 224 in which reagent 1 is accommodated are denoted as optical characteristics 1, and the measured optical characteristics of the reagent chamber 125 or 224 in which reagent 2 is accommodated are denoted as optical characteristics 2.

A control unit 320 or 420 calculates difference values by subtracting optical characteristics 2 from optical characteristics 1 and calculates the concentration of target material using the calculated difference values. For this operation, the control unit 320 or 420 pre-stores a relationship between the concentration of target material and difference values. As an example, the relationship between the concentration of target material and difference values may be represented by the calibration curve described above with reference to FIGS. 7 to 9.

The calculated concentration of target material is displayed on a display unit 301 or 401.

According to the above-described embodiments, reliability of measurement results may be enhanced by removing or minimizing interfering effects of non-target materials on the measurement results through control of the concentration of the target material present in a sample.

As is apparent from the above description, a reagent composition, a sample test method, a microfluidic device, and a test apparatus improve reliability of measurement results by removing interfering effects of non-target materials on the measurement results through capturing of the target material present in a sample.

## Claims

1. A method of determining a concentration of a target material in a sample, the method comprising:
mixing the sample with reagent 1, said reagent 1 comprising (i) an enzyme which is activated by the target material and (ii) at least one substrate of the enzyme, thereby causing the activated enzyme to act on the substrate to produce an enzyme reaction product 1;
separately mixing the sample with reagent 2, said reagent 2 comprising (i) a same enzyme which is activated by the target material, (ii) same at least one substrate of the enzyme, and (iii) a capturing material specifically capturing the target material, thereby binding at least part of the target material to the capturing material and causing the activated enzyme to act on the substrate to produce an enzyme reaction product 2;
measuring characteristics of the enzyme reaction product 1 and of the enzyme reaction product 2;
obtaining a difference value between the measured characteristics of the enzyme reaction product 1 and the measured characteristics of the enzyme reaction product 2; and
determining the concentration of the target material included in the sample using the calculated difference value.

2. The method according to claim 1, wherein the measured characteristics are optical characteristics.

3. The method according to claim 1, wherein the measured characteristics are electrical characteristics.

4. The method according to claim 1, further comprising preparing reference concentration values by plotting the differences between the measured characteristics of the enzyme reaction product 1 employing known concentrations of the target material and the measured characteristics of the enzyme reaction product 2 employing the same known concentrations of the target material.

5. The method according to claim 4, wherein the determining comprises determining the concentration of the target material included in the sample based on the reference concentration values.

6. The method according to claim 1, wherein the sample is a biological sample, and the target material is at least one of electrolyte ions present in the biological sample.

7. The method according to claim 6, wherein the target material is at least one selected from the group consisting of magnesium ions, calcium ions, potassium ions, sodium ions, and chlorine ions.

8. The method according to claim 7, wherein the capturing material is at least one selected from the group consisting of an amino carboxylic acid, a phosphonic acid, a quinoline, a crown ether, a calix-arene, a cryptand, ethylenediaminetetraacetic acid, thio-urea, and porphyrin.

9. A reagent kit comprising:
reagent 1 comprising an enzyme which is activated by a target material present in a sample and at least one substrate of the enzyme; and
reagent 2 comprising a same enzyme which is activated by the target material, same at least one substrate of the enzyme, and a capturing material specifically capturing the target material.

10. The reagent kit according to claim 9, wherein the capturing material is at least one selected from the group consisting of an amino carboxylic acid, a phosphonic acid, a quinoline, a crown ether, a calix-arene, a cryptand, ethylenediaminetetraacetic acid, thio-urea, and porphyrin.

11. A microfluidic device comprising:
a first reagent chamber loaded with reagent 1, said reagent 1 comprising an enzyme which is activated by a target material present in a sample and at least one substrate of the enzyme;
a second reagent chamber loaded with reagent 2, said reagent 2 comprising a same enzyme which is activated by the target material, same at least one substrate of the enzyme, and a capturing material specifically capturing the target material; and
a sample inlet through which the sample is introduced.

12. The microfluidic device according to claim 11, further comprising:
a first channel to connect the first reagent chamber and the sample inlet; and
a second channel to connect the second reagent chamber and the sample inlet,
wherein the first reagent chamber and the second reagent chamber are not fluid connected to each other.

13. The microfluidic device according to claim 11, wherein the sample is a biological sample, the target material is at least one of electrolyte ions present in the biological sample, and the reagent 1 and reagent 2 are in freeze-dried form.

14. The microfluidic device according to claim 11, wherein the target material is at least one selected from the group consisting of Mg²⁺, Ca²⁺, K⁺, Na⁺, and Cl⁻.

15. The microfluidic device according to claim 11, wherein the capturing material is at least one selected from the group consisting of an amino carboxylic acid, a phosphonic acid, a quinoline, a crown ether, a calix-arene, a cryptand, ethylenediaminetetraacetic acid, thio-urea, and porphyrin.

## Patentansprüche

1. Verfahren zum Bestimmen einer Konzentration eines Zielmaterials in einer Probe, wobei das Verfahren umfasst:
das Mischen der Probe mit Reagenz 1, wobei das Reagenz 1 (i) ein Enzym, welches durch das Zielmaterial aktiviert wird, und (ii) wenigstens ein Substrat des Enzyms umfasst, wodurch das aktivierte Enzym veranlasst wird, auf das Substrat einzuwirken, um ein Enzymreaktionsprodukt 1 zu erzeugen;
das getrennte Mischen der Probe mit Reagenz 2, wobei das Reagenz 2 (i) ein gleiches Enzym, welches durch das Zielmaterial aktiviert wird, (ii) das gleiche wenigstens eine Substrat des Enzyms, und (iii) ein Einfangmaterial, welches das Zielmaterial spezifisch einfängt, umfasst, wodurch wenigstens ein Teil des Zielmaterials an das Einfangmaterial gebunden wird und das aktivierte Enzym veranlasst wird, auf das Substrat einzuwirken, um ein Enzymreaktionsprodukt 2 zu erzeugen;
das Messen von Charakteristika des Enzymreaktionsprodukts 1 und des Enzymreaktionsprodukts 2;
das Erhalten eines Differenzwerts zwischen den gemessenen Charakteristika des Enzymreaktionsprodukts 1 und den gemessenen Charakteristika des Enzymreaktionsprodukts 2; und
das Bestimmen der Konzentration des in der Probe enthaltenen Zielmaterials unter Verwendung des berechneten Differenzwerts.

2. Verfahren gemäß Anspruch 1, wobei die gemessenen Charakteristika optische Charakteristika sind.

3. Verfahren gemäß Anspruch 1, wobei die gemessenen Charakteristika elektrische Charakteristika sind.

4. Verfahren gemäß Anspruch 1, außerdem umfassend das Herstellen von Referenzkonzentrationswerten durch Auftragen der Differenzen zwischen den gemessenen Charakteristika des Enzymreaktionsprodukts 1 unter Einsatz von bekannten Konzentrationen des Zielmaterials und den gemessenen Charakteristika des Enzymreaktionsprodukts 2 unter Einsatz der gleichen bekannten Konzentrationen des Zielmaterials.

5. Verfahren gemäß Anspruch 4, wobei das Bestimmen das Bestimmen der Konzentration des in der Probe enthaltenen Zielmaterials auf der Basis der Referenzkonzentrationswerte umfasst.

6. Verfahren gemäß Anspruch 1, wobei die Probe eine biologische Probe ist und das Zielmaterial wenigstens eines der in der biologischen Probe vorhandenen Elektrolytionen ist.

7. Verfahren gemäß Anspruch 6, wobei das Zielmaterial wenigstens eines, ausgewählt aus der Gruppe bestehend aus Magnesiumionen, Calciumionen, Kaliumionen, Natriumionen und Chlorionen, ist.

8. Verfahren gemäß Anspruch 7, wobei das Einfangmaterial wenigstens eines, ausgewählt aus der Gruppe bestehend aus einer Aminocarbonsäure, einer Phosphonsäure, einem Chinolin, einem Kronenether, einem Calixaren, einem Kryptanden, Ethylendiamintetraessigsäure, Thioharnstoff und Porphyrin, ist.

9. Reagenzkit umfassend:
Reagenz 1 umfassend ein Enzym, welches durch ein in einer Probe vorhandenes Zielmaterial aktiviert wird, und wenigstens ein Substrat des Enzyms; und
Reagenz 2 umfassend ein gleiches Enzym, welches durch das Zielmaterial aktiviert wird, das gleiche wenigstens eine Substrat des Enzyms, und ein Einfangmaterial, welches das Zielmaterial spezifisch einfängt.

10. Reagenzkit gemäß Anspruch 9, wobei das Einfangmaterial wenigstens eines, ausgewählt aus der Gruppe bestehend aus einer Aminocarbonsäure, einer Phosphonsäure, einem Chinolin, einem Kronenether, einem Calixaren, einem Kryptanden, Ethylendiamintetraessigsäure, Thioharnstoff und Porphyrin, ist.

11. Mikrofluidische Vorrichtung umfassend:
eine erste Reagenzkammer, die mit Reagenz 1 gefüllt ist, wobei das Reagenz 1 ein Enzym, welches durch ein in einer Probe vorhandenes Zielmaterial aktiviert wird, und wenigstens ein Substrat des Enzyms umfasst;
eine zweite Reagenzkammer, die mit Reagenz 2 gefüllt ist, wobei das Reagenz 2 ein gleiches Enzym, welches durch das Zielmaterial aktiviert wird, das gleiche wenigstens eine Substrat des Enzyms und ein Einfangmaterial, welches das Zielmaterial spezifisch einfängt, umfasst; und
einen Probeneinlass, durch den die Probe eingeführt wird.

12. Mikrofluidische Vorrichtung gemäß Anspruch 11, außerdem umfassend:
einen ersten Kanal zum Verbinden der ersten Reagenzkammer und des Probeneinlasses; und
einen zweiten Kanal zum Verbinden der zweiten Reagenzkammer und des Probeneinlasses,
wobei die erste Reagenzkammer und die zweite Reagenzkammer nicht in einer Fluidverbindung miteinander stehen.

13. Mikrofluidische Vorrichtung gemäß Anspruch 11, wobei die Probe eine biologische Probe ist, das Zielmaterial wenigstens eines der in der biologischen Probe vorhandenen Elektrolytionen ist, und das Reagenz 1 und Reagenz 2 in gefriergetrockneter Form vorliegen.

14. Mikrofluidische Vorrichtung gemäß Anspruch 11, wobei das Zielmaterial wenigstens eines, ausgewählt aus der Gruppe bestehend aus Mg²⁺, Ca²⁺, K⁺, Na⁺ und Cl⁻, ist.

15. Mikrofluidische Vorrichtung gemäß Anspruch 11, wobei das Einfangmaterial wenigstens eines, ausgewählt aus der Gruppe bestehend aus einer Aminocarbonsäure, einer Phosphonsäure, einem Chinolin, einem Kronenether, einem Calixaren, einem Kryptanden, Ethylendiamintetraessigsäure, Thioharnstoff und Porphyrin, ist.

## Revendications

1. Procédé de détermination d'une concentration d'un matériau cible dans un échantillon, le procédé consistant à:
mélanger l'échantillon avec le réactif 1, ledit réactif 1 comprenant (i) une enzyme qui est activée par le matériau cible et (ii) au moins un substrat de l'enzyme, provoquant ainsi l'action de l'enzyme activée sur le substrat pour obtenir un produit de réaction enzymatique 1;
mélanger séparément l'échantillon avec le réactif 2, ledit réactif 2 comprenant (i) une même enzyme qui est activée par le matériau cible, (ii) le même au moins un substrat de l'enzyme, et (iii) un matériau de capture capturant spécifiquement le matériau cible liant ainsi au moins une partie du matériau cible au matériau de capture et provoquant l'action de l'enzyme activée sur le substrat pour obtenir un produit de réaction enzymatique 2;
mesurer les caractéristiques du produit de réaction enzymatique 1 et du produit de réaction enzymatique 2;
obtenir une valeur de différence entre les caractéristiques mesurées du produit de réaction enzymatique 1 et les caractéristiques mesurées du produit de réaction enzymatique 2; et
déterminer la concentration du matériau cible inclus dans l'échantillon en utilisant la valeur de différence calculée.

2. Procédé selon la revendication 1, dans lequel les caractéristiques mesurées sont des caractéristiques optiques.

3. Procédé selon la revendication 1, dans lequel les caractéristiques mesurées sont des caractéristiques électriques.

4. Procédé selon la revendication 1, consistant en outre à préparer des valeurs de concentration de référence en traçant les différences entre les caractéristiques mesurées du produit de réaction enzymatique 1 en employant des concentrations connues du matériau cible et les caractéristiques mesurées du produit de réaction enzymatique 2 en employant les mêmes concentrations connues du matériau cible.

5. Procédé selon la revendication 4, dans lequel la détermination consiste à déterminer la concentration de la matière cible incluse dans l'échantillon sur la base des valeurs de concentration de référence.

6. Procédé selon la revendication 1, dans lequel l'échantillon est un échantillon biologique, et le matériau cible est au moins l'un des ions d'électrolyte présents dans l'échantillon biologique.

7. Procédé selon la revendication 6, dans lequel le matériau cible est au moins un matériau choisi dans le groupe constitué par les ions de magnésium, les ions de calcium, les ions de potassium, les ions de sodium et les ions de chlore.

8. Procédé selon la revendication 7, dans lequel le matériau de capture est au moins un matériau choisi dans le groupe consistant en un acide aminocarboxylique, un acide phosphonique, une quinoléine, un éther couronne, un calixarène, un cryptand, l'acide éthylènediaminetétraacétique, la thio-urée, et la porphyrine.

9. Kit de réactif comprenant:
le réactif 1 comprenant une enzyme qui est activée par un matériau cible présent dans un échantillon et au moins un substrat de l'enzyme; et
le réactif 2 comprenant une même enzyme qui est activée par le matériau cible, le même au moins un substrat de l'enzyme, et un matériau de capture capturant spécifiquement le matériau cible.

10. Kit de réactif selon la revendication 9, dans lequel le matériau de capture est au moins un matériau choisi dans le groupe consistant en un acide aminocarboxylique, un acide phosphonique, une quinoléine, un éther couronne, un calixarène, un cryptand, l'acide éthylènediaminetétraacétique, la thio-urée, et la porphyrine.

11. Dispositif microfluidique comprenant:
une première chambre de réactif remplie avec le réactif 1, ledit réactif 1 comprenant une enzyme qui est activée par un matériau cible présent dans un échantillon et au moins un substrat de l'enzyme;
une seconde chambre de réactif remplie avec le réactif 2, ledit réactif 2 comprenant une même enzyme qui est activée par le matériau cible, le même au moins un substrat de l'enzyme, et un matériau de capture capturant spécifiquement le matériau cible; et
une entrée d'échantillon à travers laquelle l'échantillon est introduit.

12. Dispositif microfluidique selon la revendication 11, comprenant en outre:
un premier canal pour connecter la première chambre de réactif et l'entrée d'échantillon; et
un second canal pour connecter la seconde chambre de réactif et l'entrée d'échantillon, dans lequel la première chambre de réactif et la seconde chambre de réactif ne sont pas reliées l'une à l'autre par un fluide.

13. Dispositif microfluidique selon la revendication 11, dans lequel l'échantillon est un échantillon biologique, le matériau cible est au moins l'un des ions électrolytes présents dans l'échantillon biologique, tandis que le réactif 1 et le réactif 2 sont sous forme lyophilisée.

14. Dispositif microfluidique selon la revendication 11, dans lequel le matériau cible est au moins un choisi dans le groupe consistant en Mg²⁺, Ca²⁺, K⁺, Na⁺, et Cl⁻.

15. Dispositif microfluidique selon la revendication 11, dans lequel le matériau de capture est au moins un matériau choisi dans le groupe consistant en un acide aminocarboxylique, un acide phosphonique, une quinoléine, un éther couronne, un calixarène, un cryptand, l'acide éthylènediaminetétraacétique, la thio-urée et la porphyrine.
